# EUROPEAN PATENT SPECIFICATION

(11) **EP 1 900 365 B1**
(45) Date of publication and mention of the grant of the patent: **30.03.2011**
(21) Application number: 07118561.5
(22) Date of filing: 06.02.2004
(51) Int. Cl.: A61K 31/215, A61P 29/00, A61P 9/10

(54) **Endocannabinoid-like compounds and their use for treating dermatitis**
Endocannabinoid-ähnliche Verbindungen und deren Verwendung zur Behandlung von Dermatitis
Composés de type endocannabinoïde et leur utilisation pour traiter la dermatite

(30) Priority: 07.02.2003 IT MI20030210
(43) Date of publication of application: 19.03.2008
(62) Divisional of application: 04708730.9
(73) Proprietor: Research & Innovation S.p.A., Padua (IT)
(72) Inventor: Leon, Alberta, 35142 Padova (IT); Dalle Carbonare, Maurizio, 35128 Padova (IT); Berto, Fiorenzo, 35025 Cartura (IT); Fusco, María, 35125 Padova (IT); Del Giudice, Elda, Via Svizzera, 16 , 35127 Padova (IT); Terrazzino, Salvatore, 22100 Como (IT); Facchinetti, Fabrizio, 37011 Bardolino (IT); D´Arrigo, Antonello, 35128 Padova (IT); Fabris, Michele, 35031 Abano Terme (IT)
(74) Representative: Gervasi, Gemma

(56) References cited:
- WO-A-02/24630
- WO-A-02/083059
- WO-A-03/006007
- WO-A-03/059366
- US-A- 4 049 824
- US-A- 5 231 087
- US-A- 5 409 693

## Description

### FIELD OF APPLICATION

The present invention concerns the use of amide fatty acyl- derivatives with cannabinoid-like activity for the preparation of compositions for the prevention or treatment of human or animal pathological conditions and disorders in which compounds with cannabinoid or endocannabinoid-like properties may be useful, namely dermatitis.

### STATE OF ART

Cannabinoids, such as delta-9-tetrahydrocannabinol (Δ-9-THC), the main active constituent of Cannabis sativa, are long known to possess, in addition to psychoactive effects, also beneficial pharmacological properties of potential therapeutic interest in the prevention or treatment of many pathological conditions and diseases (Ameri A., 1999 Progress in Neurobiol. 58: 315-348). Ever increasing evidences indicate that cannabinoids, such as cannabidiol or other Δ-9-THC derivatives, exert anti-inflammatory and anti-pain effects in different acute and chronic inflammatory states, including those of autoimmune nature, such as arthritis and rheumatoid arthritis, inflammatory pain, radiculopathies, asthma, ulcerous colitis, and dermatitis (Ameri A. 1999 ref. cit.). Interestingly, recent evidences also support the potential therapeutic use of Δ-9-THC derivatives in Multiple Sclerosis (MS) not only for their anti-inflammatory effect but also for their anti-spastic action observed in experimental models of MS (experimental allergic encephalitis, EAE), a result, in turn, suggestive of their capability to modulate motor control (Baker D. et. al. 2000, Nature 404, 84- 87; Ameri A. 1999 ref. cit.). Moreover, in view of the neuroprotective and anti-convulsant effects of Δ-9-THC derivatives, the potential therapeutic application of cannabino-mimetic derivatives has also been postulated to be of utility in stroke as well as in spinal or cerebral trauma. In addition, their use, either alone or in association with other drugs (e.g. opiods), has been hypothesized to be of utility for treatment also of other disorders affecting the central nervous system (CNS), particularly chronic neuropathic pain (Mas-Nieto M. et al. 2001, Brit. J. Pharmacol. 132: 1809-16).

Also of noteworthy is that the use of cannabinoid derivatives has, in view of their bronchodilator and anti-hypertensive actions, been suggested to be of utility in conditions associated with respiratory failure, cardio-vascular deficiencies and hypertension (Ameri A., 1999 ref. cit.), while their ability in modulating cellular death (anti-proliferative and pro-apoptotic effects) is suggestive of their potential utility in cancer treatment (Ameri A., 1999 ref. cit.). Furthermore, beneficial use of cannabinoids has been reported in disorders associated with loss of appetite or nausea; indeed, it has been shown that Δ-9-THC and its derivatives stimulate appetite in patients affected by AIDS syndrome in their terminal phase (Ameri A., 1999 ref. cit.) as well as reduce the nausea and emesis occurring following chemo- therapeutic treatment in oncological patients (Ameri A., 1999 ref. cit.). Finally, cannabinoid-like molecules such as palmitoylethanolamide (PEA) have been shown to inhibit the release of pro-inflammatory mediators from mast cells, suggesting their potential involvement in the modulation of allergic reactions (Facci L. et al. 1995 Proc. Natl. Acad. Sci. USA 92: 3376-80 ).

However, despite these and other evidences suggestive of a therapeutical potential of Δ-9-THC and its derivatives in a wide variety of different pathological conditions, to date the only approved clinical use of these substances is for the lowering of the intra-ocular pressure in subjects with glaucoma (Ameri A., 1999 ref. cit.). While this is, on one hand, suggestive of intrinsic difficulties in the comprehension and development of a new pharmacology based on notions deriving from the cannabinergic field, it is nevertheless noteworthy that the discovery in recent years of specific cannabinoid receptors in different species and tissues, together with the occurrence of endogenously-occurring molecules, termed endocannabinoids, capable of exerting cannabino-mimetic effects following binding to these receptors, has spurred much interest in the area of the "cannabinoid pharmacology", the ultimate objective of which is the development of new beneficial drug strategies and molecules of utility in one or more of the pathological conditions exemplified above.

To date, two different cannabinoid receptors have been identified: the so-called central CB1 receptor predominantly expressed in the CNS as well as in some peripheral tissues (Piomelli D. et al. 2000, TIPS 21: 218-24); the so-called peripheral CB2 receptor, identified in 1993, expressed in peripheral tissues, mainly in different immune cells, but not in the adult normal CNS (Piomelli D. et al. 2000). However, while there is today ever increasing experimental evidences in support of the existence of other cannabinoid receptors, these receptors have yet to be identified or fully characterised (Wiley J.L., Martin B.R. 2002, Chem. Phys. Lipids 121: 57-63).

On the other hand, the first endogenous molecule able to bind cannabinoid receptors was isolated from pig brain in 1992: this molecule, named anandamide, is N-arachidonylethanolamine, i.e. the amide between arachidonic acid and ethanolamine, now known to occur in cell membranes; its identification and capability to bind to the known CB receptors has, together with other evidences, led to the suggestion that N-acylamides found in tissues or cells may act as endogenous cannabino-mimetic mediators (Martin et al. 1999, Life Sci. 65, 573-595). Thereafter, an intermediate of mono-acylglycerol metabolism, namely 2-arachidonylglycerol, with cannabino-mimetic activity and affinity for cannabinoid receptors has also been isolated from mammalian tissues (Martin et al. 1999 ref. cit.). More recently, in addition, another endogeneously-occuring molecule, similar to 2-arachidonoylglycerol (2-AG) but with an ether link between arachidonic radical and C2 of glycerol, possessing endocannabinoid activity was isolated and characterized (Hanus L. et al. 2001, Proc. Natl. Acad. Sci. USA, 98, 3662-5). Like anandamide, both these fatty acid derivatives bind, although with different affinity, to both CB1 and CB2 receptors (Martin et al. 1999 ref. cit.). However, while this is in analogy to that of plant-derived cannabinoids such as Δ-9-THC or its synthetic derivatives, ever increasing evidences demonstrate that anandamide is, unlike these compounds, also able to bind to the vanilloid receptor VR1 (De Petrocellis et al. 2000, Chem. Phys Lipids, 108, 191-209). Indeed, while there is no evidence to date that amino-alkyl-indoles, such as WIN 55.212, bind to VR1 receptors, they nevertheless interact, in addition to cannabinoid receptors, also with other known receptors such as 5-HT3a (Barann M. et al 2002, Brit. J. Pharmacol., 137, 589-96). Thus, although endocannabinoids, like plant-derived cannabinoids, have been reported to bind to CB receptors, it cannot be excluded that endocannabinoids may interact with other receptors or, alternatively, via other mechanisms e.g. enzyme systems. In addition, while all this has given much impetus in research efforts aiming to better comprehend the pathophysiological and therapeutic significance of the endocannabinoid system in different tissues and organs, the discovery of the endocannabinoids has given way to attempts no longer focused solely on the development of derivatives able to interact with specific receptors (e.g. CB receptors) but, rather, able to augment the effects of the endogeneously-occurring endocannabinoids either by interfering with enzyme systems involved in their synthesis and degradation or, otherwise, in their cellular up-take and release. The following represents examples of substances that have been reported to exert cannabinoid-like activity: a) THC derivatives e.g. HU-210, CP 55940 (Patel S. e Hillard C. J. 2001, J. Pharmacol. Exp. Ther., 297, 629-37); b) aminoalkylindoles e.g. WIN 55.212 (Patel S. e Hillard C.J. 2001, ref. cit.); c) saturated or unsaturated endocannabinoid derivatives e.g. oleylethanolamide (OEA), palmitoylethanolamide (PEA), metandamide, olvanil, alvanil and NADE (Calignano A. et al. 2001, Eur. J. Pharmacol., 419, 191-198 ); d) inhibitors of the enzyme fatty acid amino-hydrolase (FAAH), e.g. AM374 (Gifford A.N. et al. 1999, Eur. J. Pharmacol., 383, 9-14); e) inhibitors of endocannabinoid uptake in cells e.g. AM404 (Giuffrida A. et al. 2001, J. Pharmacol. Exp. Ther., 298, 7-14). Moreover, cannabinoid receptor antagonists e.g. SR141716 and SR144528 have been developed (Francisco M.E. et al. 2002, J. Med. Chem., 45, 2708-19).

While the above clearly exemplifies the large amount of research resources in the area of the "cannabinoid pharmacology" today dedicated in the search of novel therapeutic drug strategies and molecules in different human pathologies, it is of noteworthy that, whilst molecules behaving as cannabinoid (CB1) receptor antagonists are currently in the clinical research phase, the other cannabinomimetic derivatives, mentioned above, are still at a pre-clinical stage of development. Indeed, notwithstanding demonstrations of their pharmacological efficacy in one or more experimental animal models of mammalian disorders, their development has, among different problems, been hampered by occurrence of unwanted central side effects correlated to their activation of central CB1 receptors. Moreover, although this has led to attempts to develop compounds devoid of CB1 agonist-like activity, most of the attempts aiming to develop a specific agonist for the peripheral CB2 receptor has been unsatisfactory. Indeed, most synthetic derivatives today available interact with both CB1 and CB2 receptors and, thus, are not devoid of central effects (Lambert D.M., Di Marzo V. 1999, Curr. Med. Chem., 6,757-73).

Finally, it is of noteworthy that, among different endocannabinoid-like compounds derived from the condensation of fatty acids with amino-alcohols, aromatic or not, synthesised, solely compounds characterised by an alcoholic or phenolic hydroxyl group have been reported to exert pharmacological effects in one or more experimental animal models, thus suggesting that this functional group is necessary for their biological and pharmacological actions. Indeed, although N-acyl-derivatives of arachidonic acid or other fatty acids (i.e. palmitic add) with primary amines, e.g. iso-propyl and n-propyl-amine, that do not possess hydroxyl group have already been described, there is today no evidence showing that these molecules exert pharmacological actions in vivo. In addition, while experimental in vitro studies have demonstrated that the arachidonyl-derivative possesses higher affinity for CB1 receptor (Martin et al. 1999 ref. cit.), and, hence, is of doubtful pharmacological interest In vivo in view of its potential potent psychoactive effects, the palmitoyl-derivatives have, in contrast, been shown to be devoid of significant CB receptor binding affinity. Thus, although these compounds have to date proven to be of some interest as useful tools in defining structure-activity relationships in vitro, to date there is no evidence in support of the possibility of pharmacological effects of interest of these or other similar compounds in vivo.

### SUMMARY

The synthesis of endocannabinoid-like molecules, aimed to obtain molecule with beneficial pharmacological activities but without the unwanted side effects of this kind of compounds, has allowed the Applicant to identify a family of molecules, that posses a cannabinoid-like activity in vivo but are devoid of cannabinoid central effect such as for example hypothermia, ipomotility and catalepsy.

The compounds, object of the present invention, comprise amidic derivatives with saturated aoyl residues linked to primary amine that, although the lack of the hydroxylic group, surprisingly maintain some of the cannabinoid effects and lose the central cannabinomimetic side effects, The finding that these substances do not activated the central CB1 receptors does not necessarily means that they cannot interact with other, not yet identified, not-CB1/2 central cannabinoid receptors.

Then, the object of the present invention is the use of endocannabinoid-like compounds, bearing amides groups, with general formula (I)

R-CO-NH-R₁ where and R and R₁ are defined

   according to the claims, for treating dermatitis, contact dermatitis and atopic dermatitis.

The endocannabinoid-like compounds of formule (I) can be used for the preparation of composition for therapeutic or preventive treatment of pathological condition or disorders that may be usefully treated by the cannabinoid/endocannabinold-like activity of these compounds, namely dermatitis, contact dermatitis and atopic dermatitis.

The Applicant has now suprisingly found that following in vitro or in vivo administration the compounds object of the present invention exhibited cannabinoid/endocannabinold-like pharmacological activity. In the in vivo experiments the compounds did not showed the unwanted central effects characteristic of cannabinoid/endocannabinoid compounds.

These compounds, devoid of unwanted psychotropic effects of cannabinoid/endocannabinoid derivatives, can be useful as drugs in pathological conditions that may undergo to clinical improvement upon administration of cannabinoid/endocannabinoid-like compound.

### DETAILED DESCRIPTION OF THE INVENTION

The aims and the advantages of the therapeutic use of the amidic derivatives obtained from saturated acylic residues condensed with primary amines, object of the present invention, in pathological conditions or disorders that may undergo to clinical Improvement upon administration of cannabinoids, endocannabinoids or similar molecules, namely dermatitis, contact dermatitis and atopic dermatitis, will be better understood in the course of the following description, The Applicant has in fact surprisingly found that after in vitro treatment of a human tumor cell line with the compounds object of the present invention, the compounds induced pro-apoptotic effects in a similar way as reported for synthetic cannabinoids (e.g. HU210, WIN 55.212, etc.) or known endocannabinoid-like molecules which are characterised by the presence of at least one hydroxy-alkylic or hydroxy-arylic group in the amine portion of the molecule (e.g. methanandamide, stearoylethanoalmide, palmitoyldopamide, etc.). Differently from synthetic cannabinoid or endocannabinoid, when administered in vivo, the compounds object of the present invention do not exert the unwanted central psychotropic effects characteristic of cannabinoid/endocannabinoid-like molecules (e.g. hypothermia). Moreover the Applicant has also found that in different in vivo models of inflammation, the treatment of the animals with the compounds object of the present invention induced a marked and significant reduction of the inflammation parameters such as oedema and plasma extravasation even using different inflammatory stimulus indicating that compounds are endowed with potent anti-nflammatory activity. These new and unexpected results shows for the first time that saturated acylic derivatives condensed with primary amines, object of the present invention, have cannabinoid/endocannabinoid-like effects without displaying the unwanted psychotropic effects of cannabinoid/endocannabinoid derivatives.

The endocannabinoid-like compounds and salts thereof of the present invention are defined by the general formula (I)
R-CO-NH-R₁

- where radical R is selected from the group formed by myristic, palmitic, stearic, arachidic, alkyl residues and R₁ has the meanings below defined; namely
- is the chain of a primary amine selected from the group formed by 2-amino-propane, cyclopropylamine.

The compounds herein described can be prepared in various ways. For example to, compounds with formula (I) can be prepared by adding the amine to the melted acid and heating untill the corrispondent amide is formed; .

The compounds object of the present invention were sythesized in our laboratory or, otherwise in few cases (e.g. stearylamide), obtained from commercial sources. Following are reported, for illustration, some examples of synthetised derivative.

Examples 3, 5, 7-18, 21-42 are reference examples.

### Example 1. Preparation of N-isopropyl-myristoyl amide

25 mmol of isopropylamine, dissolved in 30 ml of anhydrous dichloromethane, were placed in a 100 ml round bottom flask. To this solution have been added, drop-wise, through a loading funnel, 10 mmol of miristoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallized from n-hexane.

The reaction yield has been approximately 90%.

Physico-chemical properties of N-isopropyl-myristoyl amide:
- appearance: white crystalline powder
- formula: C₁₇H₃₅NO
- molecular weight: 269.47
- elemental analysis: C = 75.70% ; H = 13.09 ; N = 5.20 ; O = 5.94
- solubility in organic solvents: >10 mg/ml in DMSO and in chloroform
- solubility in water: scarcely soluble
- melting point: 90-92°C
- TLC: eluent: chloroform; R_{f} = 0.61

### Example 2. Preparation of N-isopropyl-palmitoyl amide

20 mmol of isopropylamine, dissolved in 25 ml of anhydrous dichloromethane, were placed in a 100 ml round bottom flask. To this solution have been added, dropwise, through a loading funnel, 8 mmol of palmitoylchloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 88%.

Physico-chemical properties of N-isopropyl-palmitoyl amide:
- appearance: white crystalline powder
- formula: C₁₉H₃₉NO
- molecular weight: 297.53
- elemental analysis: C = 76.83% ; H = 13.37 ; N = 4.71 ; O = 5.38
- solubility in organic solvents: >10 mg/ml in DMSO and in chloroform
- solubility in water: scarcely soluble
- melting point: 80-82°C
- TLC: eluent: chloroform; R_{f} = 0.59

### Example 3. Preparation of N-Isopropyl-palmitoleyl amide

A mix composed of palmitoleic acid (1 g ; 3.93 mmol) and isopropylamine (0.348 g ; 5.89 mmol) have been placed in a 50 ml round bottom flask closed with a refrigerator and heated to 140°C for an appropriate time. The reaction mixture was cooled and, when solid, solubilised in chloroform and anhydrated with sodium sulfate anhydrous.

After filtration, solvent was removed, under reduced pressure, using a rotary evaporator. The solid residue was purified using a home-made silica chromatographic column eluted with ethyl-acetate. Fractions containing N-Isopropyl-paimitoleyl amide were collected and the solvent was removed, under reduced pressure, using a rotary evaporator.

The reaction yield has been approximately 80%.

Physico-chemical properties of N-isopropyl-palmitoleyl amide:
- appearance: colourless liquid
- formula: C₁₉H₃₇NO
- molecular weight: 295.51
- elemental analysis: C = 77.24% ; H = 12.61 ; N = 4.75 ; O = 5.40
- solubility in organic solvents: >10 mg/ml in DMSO and in chloroform
- solubility in water: scarcely soluble
- melting point: < 20°C
- TLC: eluent: chloroform; R_{f} = 0.39

### Example 4. Preparation of N-Isopropyl-stearoyl amide

15 mmol of isopropylamine, dissolved in 20 ml of anhydrous dichloromethane, were placed in a 100 ml round bottom flask. To this solution have been added, dropwise, through a loading funnel, 7 mmol of stearoyl chloride dissolved in anhydrous dichloromethane. The reaction has been maintained at 0-4°C under continuos stirring and 90 minutes later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 85%.

Physico-chemical properties of N-isopropyl-stearoyl amide:
- appearance: white crystalline powder
- formula: C₂₁H₄₃NO
- molecular weight: 325.58
- elemental analysis: C = 77.45% ; H = 13.30 ; N = 4.30 ; O = 4.95
- solubility in organic solvents: >10 mg/ml in DMSO and in chloroform
- solubility in water: scarcely soluble
- melting point: 85-87°C
- TLC: eluent: chloroform; R_{f} = 0.52

### Example 5. Preparation of N-Isopropyl-oleyl amide

20 mmol of isopropylamine, dissolved in 25 ml of anhydrous dichloromethane, were placed in a 100 ml round bottom flask. To this solution have been added, drop-wise, through a loading funnel, 8 mmol of stearoyl chloride dissolved in anhydrous dichloromethane. The reaction has been maintained at 0-4°C under continuos stirring and 150 minutes later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 85%.

Physico-chemical properties of N-isopropyl-oleyl amide:
- appearance: colourless liquid
- formula: C₂₁ H₄₁ NO
- molecular weight: 323.57
- elemental analysis: C = 77.95% ; H = 12.77 ; N = 4.33 ; O = 4.95
- solubility in organic solvents: >10 mg/ml in DMSO and in chloroform
- solubility in water: scarcely soluble
- melting point: < 20°C
- TLC: eluent: chloroform; R_{f} = 0.56

### Example 6. Preparation of N-Isopropyl-arachidyl amide

A mix composed of arachidic anhidride (1 g ; 1.65 mmol) and isopropylamine (0.292 g ; 4.94 mmol) have been placed in a 50 ml round bottom flask closed with a refrigerator and heated to 150°C for an appropriate time. The reaction mixture was cooled and, when solid, crystallised from absolute ethanol. Solid crystals were washed three times with cold absolute ethanol and then dried under vacuum.

The reaction yield has been approximately 85%.

Physico-chemical properties of N-isopropyl-arachidyl amide:
- appearance: white crystalline powder
- formula: C₂₃H₄₇NO
- molecular weight: 353.64
- elemental analysis: C = 78.12% ; H = 13.39 ; N = 3.96 ; O = 4.53
- solubility in organic solvents: >10 mg/ml in DMSO and in chloroform
- solubility in water: scarcely soluble
- melting point: 85-87°C
- TLC: eluent: chloroform; R_{f} = 0.30

### Example 7. Preparation of N-β-phenylethyl-stearoyl amide

A mix composed of stearic acid (2.85 g ; 10 mmol) and β-phenylethylamine (1.82 g ; 15 mmol) have been placed in a 100 ml round bottom flask closed with a refrigerator and heated to 150°C for an appropriate time. The reaction mixture was cooled and, when solid, crystallised from absolute ethanol. Solid crystals were washed three times with cold absolute ethanol and then dried under vacuum.

The reaction yield has been approximately 80%.

Physico-chemical properties of N-β-phenylethyl-stearoyl amide:
- appearance: white crystalline powder
- formula: C₂₆H₄₅NO
- molecular weight: 387.65
- elemental analysis: C = 80.56% ; H = 11.70 ; N = 3.61 ; O = 4.13
- solubility in organic solvents: >10 mg/ml in DMSO and in chloroform
- solubility in water: scarcely soluble
- melting point: 89-91°C
- TLC: eluent: chloroform; R_{f} = 0.55

### Example 8. Preparation of n-propyl-palmitate

10 mmol of n-propanol, dissolved in 20 ml of anhydrous dichloromethane, were placed in a 100 ml round bottom flask and added of 1.5 ml of triethylamine. To this solution have been added, drop-wise, through a loading funnel, 10 mmol of palmitoylchloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing n-propyl-palmitate were collected and the solvent was removed, under reduced pressure, using a rotary evaporator.

The reaction yield has been approximately 80%.

Physico-chemical properties of n-propyl-palmitate:
- appearance: colourless liquid
- formula: C₁₉H₃₈O₂
- molecular weight: 298.51
- elemental analysis: C = 76.45% ; H = 12.83 ; O = 10.72
- solubility in organic solvents: >10 mg/ml in DMSO and in chloroform
- solubility in water: scarcely soluble
- melting point: < 20°C
- TLC: eluent: chloroform; R_{f} = 0.64

### Example 9. Preparation of n-propyl-stearate

7 mmol of n-propanol, dissolved in 20 ml of anhydrous dichloromethane, were placed in a 100 ml round bottom flask and added of 1.5 ml of triethylamine. To this solution have been added, drop-wise, through a loading funnel, 7 mmol of stearoylchloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing n-propyl-stearate were collected and the solvent was removed, under reduced pressure, using a rotary evaporator.

The reaction yield has been approximately 85%.

Physico-chemical properties of n-propyl-stearate:
- appearance: white crystalline powder
- formula: C₂₁H₄₂O₂
- molecular weight: 326.57
- elemental analysis: C = 77.24% ; H = 12.96 ; O = 9.80
- solubility in organic solvents: >10 mg/ml in DMSO and in chloroform
- solubility in water: scarcely soluble
- melting point: 26-28°C
- TLC: eluent: chloroform; R_{f} = 0.80

### Example 10. Preparation of iso-propyl-palmitate

10 mmol of iso-propanol, dissolved in 20 ml of anhydrous dichloromethane, were placed in a 100 ml round bottom flask and added of 1.5 ml of triethylamine. To this solution have been added, drop-wise, through a loading funnel, 10 mmol of palmitoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing iso-propyl-palmitate were collected and the solvent was removed, under reduced pressure, using a rotary evaporator.

The reaction yield has been approximately 90%.

Physico-chemical properties of iso-propyl-palmitate:
- appearance: colourless liquid
- formula: C₁₉H₃₈O₂
- molecular weight: 298.51
- elemental analysis: C = 76.45% ; H = 12.83 ; O = 10.72
- solubility in organic solvents: >10 mg/ml in DMSO and in chloroform
- solubility in water: scarcely soluble
- melting point: < 20°C
- TLC: eluent: chloroform; R_{f} = 0.82

### Example 11. Preparation of iso-propyl-stearate

7 mmol of iso-propanol, dissolved in 20 ml of anhydrous dichloromethane, were placed in a 100 ml round bottom flask and added of 1.5 ml of triethylamine. To this solution have been added, drop-wise, through a loading funnel, 7 mmol of stearoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing iso-propyl-stearate were collected and the solvent was removed, under reduced pressure, using a rotary evaporator.

The reaction yield has been approximately 90%.

Physico-chemical properties of iso-propyl-stearate:
- appearance: colourless liquid
- formula: C₂₁H₄₂O₂
- molecular weight: 326.57
- elemental analysis: C = 77.24% ; H = 12.96 ; O = 9.79
- solubility in organic solvents: >10 mg/ml in DMSO and in chloroform
- solubility in water: scarcely soluble
- melting point: < 20°C
- TLC: eluent: chloroform; R_{f} = 0.81

### Example 12. Preparation of N-hexadecyl-acetyl-amide

8.3 mmol of hexadecylamine, dissolved in 10 ml of anhydrous dichloromethane, were placed in a 50 ml round bottom flask and added of 4 mg of sodium acetate dissolved in chloroform. To this solution have been added, drop-wise, through a loading funnel, 6.5 mmol of acetic anhydride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified by crystallisation from-hexane.

The reaction yield has been approximately 85%.

Physico-chemical properties of N-hexadecyl-acetyl-amide:
- appearance: white crystalline powder
- formula: C₁₈H₃₇NO
- molecular weight: 283.50
- elemental analysis: C = 76.30% ; H = 13.16 ; N = 4.94 ; O = 5.60
- solubility in organic solvents: >10 mg/ml in DMSO and in chloroform
- solubility in water: scarcely soluble
- melting point: 72-74°C
- TLC: eluent: chloroform; R_{f} = 0.37

### Example 13. Preparation of N-octadecyl-acetyl-amide

7.5 mmol of octadecylamine, dissolved in 10 ml of anhydrous dichloromethane, were placed in a 50 ml round bottom flask and added of 4 mg of sodium acetate dissolved in chloroform. To this solution have been added, drop-wise, through a loading funnel, 6 mmol of acetic anhydride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified by crystallisation from-hexane.

The reaction yield has been approximately 90%.

Physico-chemical properties of N-octadecyl-acetyl-amide:
- appearance: white crystalline powder
- formula: C₂₀H₄₁NO
- molecular weight: 311.55
- elemental analysis: C=77.11%;H=13.26;N=4.50;O=5.13
- solubility in organic solvents: >10 mg/ml in DMSO and in chloroform
- solubility in water: scarcely soluble
- melting point: 74-76°C
- TLC: eluent: chloroform; R_{f} = 0.40

### Example 14. Preparation of N-cyclopentyl-palmitoyl amide

A mix composed of palmitic acid (1.80 g ; 7 mmol) and cyclopentylamine (0.894 g ; 10.5 mmol) have been placed in a 100 ml round bottom flask closed with a refrigerator and heated to 150°C for an appropriate time. The reaction mixture was cooled and, when solid, crystallised from absolute ethanol. Solid crystals were washed three times with cold absolute ethanol and then dried under vacuum.

The reaction yield has been approximately 88%.

Physico-chemical properties of N-cyclopentyl-palmitoyl amide:
- appearance: white crystalline powder
- formula: C₂₁H₄₄NO
- molecular weight: 326.59
- elemental analysis: C = 76.46% ; H = 13.45 ; N = 4.20 ; O = 5.89
- solubility in organic solvents: >10 mg/ml in DMSO and in chloroform
- solubility in water: scarcely soluble
- melting point: 65-68°C
- TLC: eluent: chloroform; R_{f} = 0.48

### Example 15. Preparation of N-cyclohexyl-palmitoyl amide

A mix composed of palmitic acid (2.56 g ; 10 mmol) and cyclohexylamine (1.49 g ; 15 mmol) have been placed in a 100 ml round bottom flask closed with a refrigerator and heated to 130°C for an appropriate time. The reaction mixture was cooled and, when solid, crystallised from absolute ethanol. Solid crystals were washed three times with cold absolute ethanol and then dried under vacuum.

The reaction yield has been approximately 90%.

Physico-chemical properties of N-cyclohexyl-palmitoyl amide:
- appearance: white crystalline powder
- formula: C₂₂H₄₆NO
- molecular weight: 340.62
- elemental analysis: C = 75.80% ; H = 13.20 ; N = 3.99 ; O = 7.01
- solubility in organic solvents: >10 mg/ml in DMSO and in chloroform
- solubility in water: scarcely soluble
- melting point: 73-75°C
- TLC: eluent: chloroform; R_{f} = 0.42

### Example 16. Preparation of N-cyclohexyl-stearoyl amide

A mix composed of stearic acid (2 g; 7 mmol) and cyclohexylamine (1.04 g ; 10.5 mmol) have been placed in a 100 ml round bottom flask closed with a refrigerator and heated to 130°C for an appropriate time. The reaction mixture was cooled and, when solid, crystallised from absolute ethanol. Solid crystals were washed three times with cold absolute ethanol and then dried under vacuum.

The reaction yeld has been approximately 87%.

Physico-chemical properties of N-cyclohexyl-stearoyl amide:
- appearance: white crystalline powder
- formula: C₂₄H₄₇NO
- molecular weight: 365.65
- elemental analysis: C = 77.80% ; H = 13.25 ; N = 3.79 ; O = 5.16
- solubility in organic solvents: >10 mg/ml in DMSO and in chloroform
- solubility in water: scarcely soluble
- melting point: 80-83°C
- TLC: eluent: chloroform; R_{f} = 0.35

### Example 17. Preparation of N-propyl-palmitoyl amide

25 mmol of n-propylamine, dissolved in 30 ml of anhydrous dichloromethane, were placed in a 100 ml round bottom flask. To this solution have been added, dropwise, through a loading funnel, 10 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 90%.

Physico-chemical properties of N-propyl-palmitoyl amide:
- appearance: white crystalline powder
- formula: C₁₉H₃₉NO
- molecular weight: 297.53
- elemental analysis: C = 76.10% ; H = 13.35 ; N = 4.70 ; O = 5.85
- solubility in organic solvents: >10 mg/ml in DMSO and in chloroform
- solubility in water: scarcely soluble
- melting point: 74-76°C
- TLC: eluent: chloroform; R_{f} = 0.51

### Example 18. Preparation of N- propyl-stearoyl amide

A mix composed of stearic acid (2.56 g; 10 mmol) and n-propylamine (0.88 g ; 15 mmol) have been placed in a 100 ml round bottom flask closed with a refrigerator and heated to 140°C for an appropriate time. The reaction mixture was cooled and, when solid, crystallised from absolute ethanol. Solid crystals were washed three times with cold absolute ethanol and then dried under vacuum.

The reaction yield has been approximately 89%.

Physico-chemical properties of N-propyl-stearoyl amide:
- appearance: white crystalline powder
- formula: C₂₁H₄₃NO
- molecular weight: 325.58
- elemental analysis: C = 77.00% ; H = 13.20 ; N = 4.30 ; O = 5.50
- solubility in organic solvents: >10 mg/ml in DMSO and in chloroform
- solubility in water: scarcely soluble
- melting point: 81-83°C
- TLC: eluent: chloroform; R_{f} = 0.47.

### Example 19. Preparation of N-cyclopropyl-palmitoyl amide

20 mmol of cyclopropylamine, dissolved in 25 ml of anhydrous dichloromethane, were placed in a 100 ml round bottom flask. To this solution have been added, drop-wise, through a loading funnel, 8 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 90%.

Physico-chemical properties of N-cyclopropyl-palmitoyl amide:
- appearance: white crystalline powder
- formula: C₁₉H₃₉NO
- molecular weight: 295.51
- elemental analysis: C = 77.40% ; H = 12.50 ; N = 4.76 ; O = 5.34
- solubility in organic solvents: >10 mg/ml in DMSO and in chloroform
- solubility in water: scarcely soluble
- melting point: 87-89°C
- TLC: eluent: chloroform; R_{f} = 0.43

### Example 20. Preparation of N- cyclopropyl-stearoyl amide

A mix composed of stearic acid (2.56 g ; 10 mmol) and cyclopropylamine (0.86 g ; 15 mmol) have been placed in a 100 ml round bottom flask closed with a refrigerator and heated to 130°C for an appropriate time. The reaction mixture was cooled and, when solid, crystallised from absolute ethanol. Solid crystals were washed three times with cold absolute ethanol and then dried under vacuum.

The reaction yield has been approximately 86%.

Physico-chemical properties of N-cyclopropyl-stearoyl amide:
- appearance: white crystalline powder
- formula: C₂₁H₄₁NO
- molecular weight: 323.57
- elemental analysis: C = 77.50% ; H = 12.70 ; N = 4.30 ; O = 5.50
- solubility in organic solvents: >10 mg/ml in DMSO and in chloroform
- solubility in water: scarcely soluble
- melting point: 81-83°C
- TLC: eluent: ethyl acetate; R_{f} = 0.35.

### Example 21. Preparation of cyclo-propyl-palmitate

10 mmol of cyclopropanol, dissolved in 20 ml of anhydrous dichloromethane, were placed in a 100 ml round bottom flask and added of 1.5 ml of triethylamine. To this solution have been added, drop-wise, through a loading funnel, 10 mmol of palmitoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing cyclopropyl-palmitate were collected and the solvent was removed, under reduced pressure, using a rotary evaporator.

The reaction yield has been approximately 80%.

Physico-chemical properties of cyclopropyl-palmitate:
- appearance: colourless liquid
- formula: C₁₉H₃₈O₂
- molecular weight: 298.51
- elemental analysis: C = 75.80% ; H = 12.80 ; O = 11.40
- solubility in organic solvents: >10 mg/ml in DMSO and in chloroform
- solubility in water: scarcely soluble
- melting point: < 20°C
- TLC: eluent: chloroform; R_{f} = 0.78

### Example 22. Preparation of cyclopropyl-stearate

7 mmol of cyclopropanol, dissolved in 20 ml of anhydrous dichloromethane, were placed in a 100 ml round bottom flask and added of 1.5 ml of triethylamine. To this solution have been added, drop-wise, through a loading funnel, 7 mmol of stearoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing cyclopropyl-stearate were collected and the solvent was removed, under reduced pressure, using a rotary evaporator.

The reaction yield has been approximately 85%.

Physico-chemical properties of cyclopropyl-stearate:
- appearance: colourless liquid
- formula: C₂₁H₄₂O₂
- molecular weight: 326.57
- elemental analysis: C = 77.50% ; H = 12.98 ; O = 9.52
- solubility in organic solvents: >10 mg/ml in DMSO and in chloroform
- solubility in water: scarcely soluble
- melting point: < 20°C
- TLC: eluent: chloroform; R_{f} = 0.75

### Example 23. Preparation of N-butyl-palmitoyl amide

25 mmol of butylamine, dissolved in 30 ml of anhydrous dichloromethane, were placed in a 100 ml round bottom flask. To this solution have been added, drop-wise, through a loading funnel, 10 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 93%.

Physico-chemical properties of N-butyl-palmitoyl amide:
- appearance: white crystalline powder
- formula: C₂₀H₄₁NO
- molecular weight: 311.55
- elemental analysis: C = 77.30% ; H = 13.30 ; N = 4.50 ; O = 4.90
- solubility in organic solvents: >10 mg/ml in DMSO and in chloroform
- solubility in water: scarcely soluble
- melting point: 72-74°C
- TLC: eluent: chloroform; R_{f} = 0.47

### Example 24. Preparation of N-butyl-stearoyl amide

A mix composed of stearic acid (2.56 g ; 10 mmol) and butylamine (1.10 g ; 15 mmol) have been placed in a 100 ml round bottom flask closed with a refrigerator and heated to 150°C for an appropriate time. The reaction mixture was cooled and, when solid, crystallised from absolute ethanol. Solid crystals were washed three times with cold absolute ethanol and then dried under vacuum.

The reaction yield has been approximately 89%.

Physico-chemical properties of N-butyl-stearoyl amide:
- appearance: white crystalline powder
- formula: C₂₂H₄₅NO
- molecular weight: 339.61
- elemental analysis: C = 77.70% ; H = 13.35 ; N = 4.15 ; O = 4.80
- solubility in organic solvents: >10 mg/ml in DMSO and in chloroform
- solubility in water: scarcely soluble
- melting point: 79-81°C
- TLC: eluent: chloroform; R_{f} = 0.54.

### Example 25. Preparation of N-isobutyl-palmitoyl amide

20 mmol of isobutylamine, dissolved in 25 ml of anhydrous dichloromethane, were placed in a 100 ml round bottom flask. To this solution have been added, drop-wise, through a loading funnel, 8 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 90%.

Physico-chemical properties of N-isobutyl-palmitoyl amide:
- appearance: white crystalline powder
- formula: C₂₀H₄₁NO
- molecular weight: 311.55
- elemental analysis: C = 77.20% ; H = 13.28 ; N = 4.52 ; O = 5.00
- solubility in organic solvents: >10 mg/ml in DMSO and in chloroform
- solubility in water: scarcely soluble
- melting point: 78-80°C
- TLC: eluent: chloroform; R_{f} = 0.53

### Example 26. Preparation of N-isobutyl-stearoyl amide

25 mmol of isobutylamine, dissolved in 30 ml of anhydrous dichloromethane, were placed in a 100 ml round bottom flask. To this solution have been added, drop-wise, through a loading funnel, 10 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 86%.

Physico-chemical properties of N-isobutyl-stearoyl amide:
- appearance: white crystalline powder
- formula: C₂₂H₄₅NO
- molecular weight: 339.61
- elemental analysis: C = 77.92% ; H = 13.41 ; N = 4.14 ; O = 4.53
- solubility in organic solvents: >10 mg/ml in DMSO and in chloroform
- solubility in water: scarcely soluble
- melting point: 83-85°C
- TLC: eluent: chloroform; R_{f} = 0.57

### Example 27. Preparation of N-cyclobutyl-palmitoyl amide

25 mmol of cyclobutylamine, dissolved in 30 ml of anhydrous dichloromethane, were placed in a 100 ml round bottom flask. To this solution have been added, drop-wise, through a loading funnel, 10 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 90%.

Physico-chemical properties of N-cyclobutyl-palmitoyl amide:
- appearance: white crystalline powder
- formula: C₂₀H₃₉NO
- molecular weight: 309.54
- elemental analysis: C = 77.70% ; H = 12.70 ; N = 4.54 ; O = 5.06
- solubility in organic solvents: >10 mg/ml in DMSO and in chloroform
- solubility in water: scarcely soluble
- melting point: 77-79°C
- TLC: eluent: chloroform; R_{f} = 0.48

### Example 28. Preparation of N-cyclobutyl-stearoyl amide

20 mmol of cyclobutylamine, dissolved in 25 ml of anhydrous dichloromethane, were placed in a 100 ml round bottom flask. To this solution have been added, drop-wise, through a loading funnel, 8 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 86%.

Physico-chemical properties of N-cyclobutyl-stearoyl amide:
- appearance: white crystalline powder
- formula: C₂₂H₄₃NO
- molecular weight: 337.59
- elemental analysis: C = 78.30% ; H = 12.85 ; N = 4.16 ; O = 4.69
- solubility in organic solvents: >10 mg/ml in DMSO and in chloroform
- solubility in water: scarcely soluble
- melting point: 83-85°C
- TLC: eluent: chloroform; R_{f} = 0.55

### Example 29. Preparation of n-butyl-palmitate

10 mmol of n-butanol, dissolved in 20 ml of anhydrous dichloromethane, were placed in a 100 ml round bottom flask and added of 1.5 ml of triethylamine. To this solution have been added, drop-wise, through a loading funnel, 10 mmol of palmitoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing n-butyl-palmitate were collected and the solvent was removed, under reduced pressure, using a rotary evaporator.

The reaction yield has been approximately 80%.

Physico-chemical properties of n-butyl-palmitate:
- appearance: colourless liquid
- formula: C₂₀H₄₀O₂
- molecular weight: 312.54
- elemental analysis: C = 76.90% ; H = 13.00 ; O = 10.10
- solubility in organic solvents: >10 mg/ml in DMSO and in chloroform
- solubility in water: scarcely soluble
- melting point: < 20°C
- TLC: eluent: chloroform; R_{f} = 0.56

### Example 30. Preparation of n-butyl-stearate

7 mmol of n-butanol, dissolved in 20 ml of anhydrous dichloromethane, were placed in a 100 ml round bottom flask and added of 1.5 ml of triethylamine. To this solution have been added, drop-wise, through a loading funnel, 7 mmol of stearoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing n-butyl-stearate were collected and the solvent was removed, under reduced pressure, using a rotary evaporator.

The reaction yield has been approximately 85%.

Physico-chemical properties of n-butyl-stearate:
- appearance: white crystalline powder
- formula: C₂₂H₄₄O₂
- molecular weight: 340.59
- elemental analysis: C = 76.90% ; H = 13.00 ; O = 10.10
- solubility in organic solvents: >10 mg/ml in DMSO and in chloroform
- solubility in water: scarcely soluble
- melting point: < 20°C
- TLC: eluent: chloroform; R_{f} = 0.61

### Example 31. Preparation of iso-butyl-palmitate

10 mmol of iso-butanol, dissolved in 20 ml of anhydrous dichloromethane, were placed in a 100 ml round bottom flask and added of 1.5 ml of triethylamine. To this solution have been added, drop-wise, through a loading funnel, 10 mmol of palmitoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing iso-butyl-palmitate were collected and the solvent was removed, under reduced pressure, using a rotary evaporator.

The reaction yield has been approximately 85%.

Physico-chemical properties of iso-butyl-palmitate:
- appearance: colourless liquid
- formula: C₂₀H₄₀O₂
- molecular weight: 312.54
- elemental analysis: C = 76.80% ; H = 12.89 ; O = 10.31
- solubility in organic solvents: >10 mg/ml in DMSO and in chloroform
- solubility in water: scarcely soluble
- melting point: < 20°C
- TLC: eluent: chloroform; R_{f} = 0.78

### Example 32. Preparation of iso-butyl-stearate

7 mmol of iso-butanol, dissolved in 20 ml of anhydrous dichloromethane, were placed in a 100 ml round bottom flask and added of 1.5 ml of triethylamine. To this solution have been added, drop-wise, through a loading funnel, 7 mmol of stearoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing iso-butyl-stearate were collected and the solvent was removed, under reduced pressure, using a rotary evaporator.

The reaction yield has been approximately 88%.

Physico-chemical properties of iso-butyl-stearate:
- appearance: colourless liquid
- formula: C₂₂H₄₄O₂
- molecular weight: 340.59
- elemental analysis: C = 77.60% ; H = 13.10 ; O = 9.30
- solubility in organic solvents: >10 mg/ml in DMSO and in chloroform
- solubility in water: scarcely soluble
- melting point: < 20°C
- TLC: eluent: chloroform; R_{f} = 0.80

### Example 33. Preparation of N-pentyl-palmitoyl amide

25 mmol of pentylamine, dissolved in 30 ml of anhydrous dichloromethane, were placed in a 100 ml round bottom flask. To this solution have been added, drop-wise, through a loading funnel, 10 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 90%.

Physico-chemical properties of N-pentyl-palmitoyl amide:
- appearance: white crystalline powder
- formula: C₂₁ H₄₃NO
- molecular weight: 325.58
- elemental analysis: C = 77.30% ; H = 13.40 ; N = 4.92 ; O = 4.38
- solubility in organic solvents: >10 mg/ml in DMSO and in chloroform
- solubility in water: scarcely soluble
- melting point: 74-76°C
- TLC: eluent: chloroform; R_{f} = 0.68

### Example 34. Preparation of N- pentyl-stearoyl amide

A mix composed of stearic acid (2.56 g ; 10 mmol) and pentylamine (1.10 g ; 15 mmol) have been placed in a 100 ml round bottom flask closed with a refrigerator and heated to 150°C for an appropriate time. The reaction mixture was cooled and, when solid, crystallised from absolute ethanol. Solid crystals were washed three times with cold absolute ethanol and then dried under vacuum.

The reaction yield has been approximately 85%.

Physico-chemical properties of N-pentyl-stearoyl amide:
- appearance: white crystalline powder
- formula: C₂₃H₄₇NO
- molecular weight: 353.64
- elemental analysis: C = 78.20% ; H = 13.40 ; N = 3.98 ; O = 4.42
- solubility in organic solvents: >10 mg/ml in DMSO and in chloroform
- solubility in water: scarcely soluble
- melting point: 83-85°C
- TLC: eluent: chloroform; R_{f} = 0.74.

### Example 35. Preparation of N-isopentyl-palmitoyl amide

20 mmol of isopentylamine, dissolved in 25 ml of anhydrous dichloromethane, were placed in a 100 ml round bottom flask. To this solution have been added, drop-wise, through a loading funnel, 8 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 86%.

Physico-chemical properties of N-isopentyl-palmitoyl amide:
- appearance: white crystalline powder
- formula: C₂₁H₄₃NO
- molecular weight: 325.58
- elemental analysis: C = 77.50% ; H = 13.35 ; N = 4.32 ; O = 4.83

- solubility in organic solvents: >10 mg/ml in DMSO and in chloroform
- solubility in water: scarcely soluble
- melting point: 85-87°C
- TLC: eluent: chloroform; R_{f} = 0.68

### Example 36. Preparation of N-isopentyl-stearoyl amide

25 mmol of isopentylamine, dissolved in 30 ml of anhydrous dichloromethane, were placed in a 100 ml round bottom flask. To this solution have been added, drop-wise, through a loading funnel, 10 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was cristallized from n-hexane.

The reaction yield has been approximately 80%.

Physico-chemical properties of N-isopentyl-stearoyl amide:
- appearance: white crystalline powder
- formula: C₂₃H₄₇NO
- molecular weight: 353.64
- elemental analysis: C = 77.90% ; H = 13.40 ; N = 3.94 ; O = 4.76
- solubility in organic solvents: >10 mg/ml in DMSO and in chloroform
- solubility in water: scarcely soluble
- melting point: 87-89°C
- TLC: eluent: chloroform; R_{f} = 0.73

### Example 37. Preparation of N-cyclopentyl-palmitoyl amide

25 mmol of cyclopentylamine, dissolved in 30 ml of anhydrous dichloromethane, were placed in a 100 ml round bottom flask. To this solution have been added, drop-wise, through a loading funnel, 10 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 87%.

Physico-chemical properties of N-cyclopentyl-palmitoyl amide:
- appearance: white crystalline powder
- formula: C₂₁H₄₁NO
- molecular weight: 323.57
- elemental analysis: C = 78.00% ; H = 12.90 ; N = 4.35 ; O = 4.75
- solubility in organic solvents: >10 mg/ml in DMSO and in chloroform
- solubility in water: scarcely soluble
- melting point: 83-85°C
- TLC: eluent: chloroform; R_{f} = 0.56

### Example 38. Preparation of N-cyclopentyl-stearoyl amide

20 mmol of cyclopentylamine, dissolved in 25 ml of anhydrous dichloromethane, were placed in a 100 ml round bottom flask. To this solution have been added, drop-wise, through a loading funnel, 8 mmol of palmitoyl chloride dissolved in anhydrous dichloromethane. The reaction has been maintained at 0-4°C under continuos stirring and two hours later the reaction was stopped with the addition of a 10% citric acid aqueous solution. Organic phase was separated, collected and anhydrated with anhydrous sodium sulfate and filtered. Solvent was removed, under reduced pressure, using a rotary evaporator. The solid residue was crystallised from n-hexane.

The reaction yield has been approximately 83%.

Physico-chemical properties of N-cyclopentyl-stearoyl amide:
- appearance: white crystalline powder
- formula: C₂₃H₄₅NO
- molecular weight: 351.62
- elemental analysis: C = 77.90% ; H = 12.80 ; N = 3.96 ; O = 5.34
- solubility in organic solvents: >10 mg/ml in DMSO and in chloroform
- solubility in water: scarcely soluble
- melting point: 85-87°C
- TLC: eluent: chloroform; R_{f} = 0.61

### Example 39. Preparation of n-pentyl-palmitate

10 mmol of n-pentanol, dissolved in 20 ml of anhydrous dichloromethane, were placed in a 100 ml round bottom flask and added of 1.5 ml of triethylamine. To this solution have been added, drop-wise, through a loading funnel, 10 mmol of palmitoy lchloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing n-pentyl-palmitate were collected and the solvent was removed, under reduced pressure, using a rotary evaporator.

The reaction yield has been approximately 80%.

Physico-chemical properties of n-pentyl-palmitate:
- appearance: colourless liquid
- formula: C₂₁H₄₂O₂
- molecular weight: 326.57
- elemental analysis: C = 76.80% ; H = 12.90 ; O = 10.30
- solubility in organic solvents: >10 mg/ml in DMSO and in chloroform
- solubility in water: scarcely soluble
- melting point: < 20°C
- TLC: eluent: chloroform; R_{f} = 0.62

### Example 40. Preparation of n-pentyl-stearate

7 mmol of n-pentanol, dissolved in 20 ml of anhydrous dichloromethane, were placed in a 100 ml round bottom flask and added of 1.5 ml of triethylamine. To this solution have been added, drop-wise, through a loading funnel, 7 mmol of stearoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing n-pentyl-stearate were collected and the solvent was removed, under reduced pressure, using a rotary evaporator.

The reaction yield has been approximately 88%.

Physico-chemical properties of n-pentyl-stearate:
- appearance: white crystalline powder
- formula: C₂₃H₄₆O₂
- molecular weight: 354.62
- elemental analysis: C = 78.50% ; H = 13.10 ; O = 8.40
- solubility in organic solvents: >10 mg/ml in DMSO and in chloroform
- solubility in water: scarcely soluble
- melting point: < 20°C
- TLC: eluent: chloroform; R_{f} = 0.60

### Example 41. Preparation of cyclopentyl-palmitate

10 mmol of cyclopentanol, dissolved in 20 ml of anhydrous dichloromethane, were placed in a 100 ml round bottom flask and added of 1.5 ml of triethylamine. To this solution have been added, drop-wise, through a loading funnel, 10 mmol of palmitoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing cyclopentyl-palmitate were collected and the solvent was removed, under reduced pressure, using a rotary evaporator.

The reaction yield has been approximately 85%.

Physico-chemical properties of cyclopentyl-palmitate:
- appearance: colourless liquid
- formula: C₂₁H₄₀O₂
- molecular weight: 324.55
- elemental analysis: C = 77.80% ; H = 12.48 ; O = 9.72
- solubility in organic solvents: >10 mg/ml in DMSO and in chloroform
- solubility in water: scarcely soluble
- melting point: < 20°C
- TLC: eluent: chloroform; R_{f} = 0.55

### Example 42. Preparation of cyclopentyl-stearate

7 mmol of cyclopentanol, dissolved in 20 ml of anhydrous dichloromethane, were placed in a 100 ml round bottom flask and added of 1.5 ml of triethylamine. To this solution have been added, drop-wise, through a loading funnel, 7 mmol of stearoyl chloride dissolved in anhydrous dichloromethane. The reaction has been maintained at 0-4°C under continuos stirring and 2 hrs later the reaction was stopped. Solvent was removed, under reduced pressure, using a rotary evaporator. The residue was purified using a home-made silica chromatographic column eluted with chloroform. Fractions containing cyclopentyl-stearate were collected and the solvent was removed, under reduced pressure, using a rotary evaporator.

The reaction yield has been approximately 90%.

Physico-chemical properties of cyclopentyl-stearate:
- appearance: colourless liquid
- formula: C₂₃H₄₀O₂
- molecular weight: 348.57
- elemental analysis: C = 78.80% ; H = 11.48 ; O = 9.72
- solubility in organic solvents: >10 mg/ml in DMSO and in chloroform
- solubility in water: scarcely soluble
- melting point: < 20°C
- TLC: eluent: chloroform; R_{f} = 0.54

### A) In vitro cannabinoid-like effects.

In all the following tests, only examples 1, 2, 4, 6, 19, 20 are part of the invention.

### Evaluation of cellular viability by mean of MTT assay.

### Experimental model

Jurkat, an acute T-lymphoblastic leukemia cell line generated from a 14-year-old male, have been used. Cell culture have been cultured in RPMI-1640 culture medium containing 10% FBS.

### Experimental schedule

Jurkat cells were cultured at 1x10⁵ cells/ml in RPMI 1640 medium supplemented with 10% fetal bovine serum (FBS), 2mM L-glutamine and 100 U/ml penicillin + 100 ug/ml streptomycin.

To detect drug effect in vitro, MTT [3-(4,5-dimethylthiazol-2-yl)-2,5-diphenyltetrazolium bromide] assay was used as described (Carmichael J et al., 1987 Cancer Res. 47, 936-42). Briefly, cells were seeded in triplicate on 96-well culture plates at a density of 20000 cells/well. The drugs were added in order to have the final concentration of 10 µM (1 µl of a solution of 10mM in DMSO) and incubated for 18-24 hr. Then MTT (0.5 mg/ml) was added to each well, and plates were incubated for an additional 1 to 3 hr in the dark at 37 °C. After this time the MTT solution was aspirated from the wells and 0.1 ml of DMSO was added. The plates were allowed to stand for 10 min, and then the absorbance was determined in a SpectraCount (Packard) microplate reader at 570 nm. The drug effect was determined by comparing absorbances of treated and untreated cells.

### Results.

The results concerning the cellular death are reported in the Table 1.

**Table 1. Cellular viability inhibition in Jurkat cells.**

| MOLECULES | Growth inhibition (% of control) | MOLECULES | Growth inhibition (% of control) |
|---|---|---|---|
| CONTROL | 0 | Example 27 | 29.2 |
| Example 1 | 55.0 | Example 28 | 31.6 |
| Example 2 | 55.6 | Example 29 | 15.2 |
| Example 3 | 60.5 | Example 30 | 11.3 |
| Example 4 | 45.3 | Example 31 | 8.7 |
| Example 5 | 66.6 | Example 32 | 6.5 |
| Example 6 | 41.0 | Example 33 | 33.3 |
| Example 7 | 47.3 | Example 34 | 34.2 |
| Example 8 | 22.2 | Example 35 | 29.2 |
| Example 9 | 18.7 | Example 36 | 30.2 |
| Example 10 | 20.4 | Example 37 | 32.1 |
| Example 11 | 17.6 | Example 38 | 31.8 |
| Example 12 | 64.8 | Example 39 | 6.6 |
| Example 13 | 67.2 | Example 40 | 7.3 |
| Example 14 | 52.6 | Example 41 | 7.6 |
| Example 15 | 63.2 | Example 42 | 7.2 |
| Example 16 | 18.2 | Starylamide | 35.2 |
| Example 17 | 21.4 | Oleylamide | 22.1 |
| Example 18 | 17.3 | Palmitoylamide | 33.9 |
| Example 19 | 56.2 | ANANDAMIDE | 89.0 |
| Example 20 | 64.2 | 2-ARACHIDONYL GLYCEROL | 18.8 |
| Example 21 | 22.1 | HU-210 | 89.0 |
| Example 22 | 13.2 | CP55940 | 89.6 |
| Example 23 | 36.0 | WIN 55.212 | 79.2 |
| Example 24 | 32.1 | JWH 133 | 2.0 |
| Example 25 | | SR141716 | 87.0 |
| Example 26 | | SR144528 | 8.1 |

The results obtained have shown that the molecules object of present invention, similar to natural or synthetic cannabinoids, are able to induce cellular death in vitro.

### B) In vivo cannabinoid-like effects.

### Experimental model

The Arthus model (RPA) is a model of immune-complex mediated tissue damage that involves the passive subcutaneous introduction of an antibody and the systemic administration of the corresponding antigen. This inflammatory model has been used to test the in vivo activity of the synthesised molecules.

The reverse passive Arthus reaction is induced, in the mouse ear pinna, by the subcutaneous injection of an excess of IgG anti human albumin followed by i.v. adiministration of the corresponding Ag (i.e. human albumin) together with Evans blue, 2 mg for each mouse, for the evaluation of extravasation.

The tested molecules were solubilised in DMSO, cremophor and PBS in a ratio of 1:1:18 and administered usually by intraperitonelly at the dosage of 10 mg/Kg 1 hour before the induction of anafilactic reaction. The animals were sacrificed 3 hours after the molecule administration.

The oedema and the extravasations are evaluated tree hours after the i.v. administration of the antigen. The oedema is evaluated as the increase of weight of lesioned ear area dissected by a 8 mm biopsy punch as compared to the contralateral unlesioned auricular tissues. The extravasation is measured determining the Evans blue density at 620 nm in the supernatants deriving from ear homogenates following centrifugation at 10000 x g for 20 min.

### Results

The administration of cannabinoids, endocannabinoids and molecules that are object of the present invention cause a significant reduction of the development of oedema induced by PCA (see table 2).

**TABLE 2. Oedema formation in mouse ear following PCA reaction in animals that have been treated with cannabinoids, endocannabinoids or molecules that are object of the present invention.**

| | Oedema (mg) | P |
|---|---|---|
| Vehicle | 49,8 ± 1,2 | |
| Naive | 23,7 ± 1,2 | < 0.0001 |
| Example 1 | 39,9 ± 2,1 | < 0.001 |
| Example2 | 31,8 ± 1,8 | < 0.0001 |
| Example 4 | 26,5 ± 0,9 | < 0.0001 |
| Example 12 | 41,5 ± 1,9 | < 0.1 |
| Example 13 | 43,3 ± 0.6 | < 0.1 |
| Example 14 | 39,5 ± 0.8 | < 0.1 |
| Example 15 | 47,5 ± 4,2 | < 0.1 |
| Example 16 | 48,2 ± 1.8 | < 0.1 |
| Example 17 | 32,3 ± 2.7 | < 0.01 |
| Example 18 | 33,3 ± 0.2 | < 0.001 |
| Example 19 | 31,8 ± 3.6 | < 0.001 |
| Example 20 | 33,1 ± 0.9 | < 0.001 |
| Example 23 | 35,2 ± 1.7 | < 0.01 |
| Example 24 | 33,6 ± 1.2 | < 0.01 |
| Example 25 | 42,4 ± 2.4 | < 0.1 |
| Example 26 | 40,3 ± 0.7 | < 0.1 |
| Example 27 | 38,5 ± 0.9 | < 0.1 |
| Example 28 | 40,6 ± 2.6 | < 0.1 |
| Example 33 | 43,9 ± 2.4 | < 0.1 |
| Example 34 | 42,5 ± 3.5 | < 0.1 |
| Example 35 | 38,3 ± 1.8 | < 0.1 |
| Example 36 | 33,7 ± 3.2 | < 0.01 |
| Example 37 | 36,2 ± 0.9 | < 0.01 |
| Example 38 | 32,3 ± 3.3 | < 0.01 |
| Starylamide | 30,8 ± 0.5 | < 0.01 |
| Oleylamide | 43,5 ± 2.6 | < 0.1 |
| Palmitoylamide | 33,2 ± 0.7 | < 0.01 |
| ANANDAMIDE | 35,5 ± 2,7 | < 0.001 |
| WIN 55.212 | 33,7 ± 4,6 | < 0.05 |

Together with oedema reduction, cannabinoids, endocannabinoids and molecules that are object of the present invention, when administered i.p. also induce a significant reduction of the development of extravasation induced by PCA (see table 3).

**TABLE 3. Extravasation development in mouse ear following PCA reaction in animals that have been treated with cannabinoids, endocannabinoids or molecules that are object of the present invention.**

| | Extravasation | p | Riduction % |
|---|---|---|---|
| Vehicle | 0,90±0,06 | | 0 |
| Naïve | 0,04±0,01 | < 0.0001 | |
| Example 1 | 0,57±0,03 | < 0.05 | 36.7 |
| Example2 | 0,37±0,07 | < 0.0001 | 58.9 |
| Example 4 | 0,20±0,03 | < 0.0001 | 77.8 |
| Example 12 | 0,72±0,03 | > 0.1 | 20.0 |
| Example 13 | 0,74±0,07 | > 0.1 | 17.2 |
| Example 14 | 0,52±0,06 | < 0.05 | 32.3 |
| Example 15 | 0,58±0,16 | > 0.1 | 35.5 |
| Example 16 | 0,66±0,09 | > 0.1 | 26,7 |
| Example 17 | 0,52±0,12 | > 0.1 | 32.3 |
| Example 18 | 0,54±0,03 | < 0.05 | 40.0 |
| Example 19 | 0,32±0,02 | < 0.001 | 64.6 |
| Example 20 | 0,35±0,06 | < 0.001 | 61.2 |
| Example 23 | 0,59±0,08 | < 0.05 | 34.5 |
| Example 24 | 0,55±0,07 | < 0.05 | 38.9 |
| Example 25 | 0,72±0,07 | > 0.1 | 20.0 |
| Example 26 | 0,81±0,05 | > 0.1 | 10.0 |
| Example 27 | 0,39±0,11 | < 0.001 | 56.7 |
| Example 28 | 0,31±0,12 | < 0.001 | 65.6 |
| Example 33 | 0,72±0,06 | > 0.1 | 20.0 |
| Example 34 | 0,62±0,08 | > 0.1 | 31.2 |
| Example 35 | 0,58±0,09 | < 0.05 | 35.6 |
| Example 36 | 0,52±0,05 | < 0.05 | 42.7 |
| Example 37 | 0,64±0,13 | < 0.001 | 52.5 |
| Example 38 | 0,51±0,14 | < 0.05 | 43.4 |
| Starylamide | 0,31±0,08 | < 0.001 | 65.6 |
| Oleylamide | 0,65±0,12 | > 0.1 | 21.8 |
| Palmitoylamide | 0,35±0,05 | < 0.001 | 61.2 |
| ANANDAMIDE | 0,65±0,09 | > 0.1 | 27.8 |
| WIN 55.212 | 0,51 ±0,02 | > 0.01 | 43.5 |

The administration of synthetic cannabinoid such as Win 55,212 or the endocannabinoid Anandamide produces a drastic reduction of the rectal temperature while no alteration was induced by the molecules that are object of the present invention (see table 4).

**TABLE 4. Rectal temperature in animals that have been treated with cannabinoids, endocannabinoids or molecules that are object of the present invention. Note a profound hypothermia induced by synthetic cannabinoid such as Win 55,212 or the endocannabinoid Anandamide while no alteration was induced by molecules that are object of the present invention.**

| | ΔT (°C) |
|---|---|
| Vehicle | 0,1 ± 0,2 |
| Naive | 0,1 ± 0,2 |
| Example 1 | 0,2 ± 0,3 |
| Example2 | 0,1 ± 0,1 |
| Example 4 | 0,2 ± 0,1 |
| Example 12 | 0,2 ± 0,2 |
| Example 15 | 0,1 ± 0,1 |
| Example 25 | 0,3 ± 0,1 |
| Example 27 | 0,1 ± 0,1 |
| Example 34 | 0,1 ± 0,1 |
| Example 35 | 0,3 ± 0,2 |
| Example 36 | 0,1 ± 0,1 |
| Example 38 | 0,2 ± 0,2 |
| Stearylamide | 0,3 ± 0,2 |
| Palmitoylamide | 0,2 ± 0,1 |
| Example | 0,2 ± 0,2 |
| ANANDAMIDE | -4,2 ± 1,2 |
| WIN 55.212 | -5,1 ± 1,3 |

The results obtained in vivo demonstrate that the administration of saturated or acyl-amide derivatives are able to significantly reduce both oedema and extravasation formation that occur following PCA reaction. It is important to point out that these results demonstrate that this class of molecules are endowed with anti-inflammatory activity. More interestingly is that these effects seems to be mediated by a peripheral mechanism since the tested molecules, at the used concentration, did not induce central effect.

The present invention describe a cannabinoid-like effect of the selected molecules on a peripheral receptor, probably an unidentified cannabinoid receptor, different from CB2 since the CB2 selective agonist JWH133 is unable to induce cellular death in vitro and to reduce oedema and extravasation in vivo. The lack of involvement of CB2 receptor is also supported by the finding the CB2 selective antagonist SR144528 did not antagonize the anti-inflammatory effect of cannabinoid, endocannabinoid or molecules object of the present invention. These data support the increasing body of evidences suggesting the existence of unidentified CB receptors.

The compounds object of the present invention can be used alone or in combination with other drugs to treat human or animal pathological conditions in which the endocannabinoid system may be involved. On the basis of the in vitro and in vivo cannabinoid-like effects shown by the compounds object of the present invention and the absence of CB1-mediated central effects, these substances might be used for the preparation of pharmaceutical compositions for the prevention or treatment alone or in combination with other specific therapeutic agents of specific pathological state or disorder. Molecules with cannabinoid-like activity might in fact be association with anti epileptic, neuroleptic, atypical neuroleptic, anti depressant, dopaminergic, dopamine agonists, GABA agonist drugs; moreover this molecules, alone or in combination with specific drugs, might be useful for control of weight gain or to improve memory deficits; finally they can be used as anti inflammatory and anti pain and thus in association with opiods, salicilates, pyrazoles, aryl-anthranilates, aryl-propyonates, aryl-acetates, oxicams, pyrazolones, glucocorticoids, cox2-inhibitors, nimesulide and para-aminophenoles. Pathological conditions that may take advantage from an endocannabinoid-like effect include:
- given their anti-inflammatory and anti pain effect they may be useful in treating arthritis, including rheumatoid arthritis, or other chronic inflammatory conditions as well as inflammatory conditions of autoimmune origin or not (e.g. chronic pain, radiculopathies, asthma, rheumatoid arthritis, ulcerous colitis, dermatitis, etc.);
- given the neuro-protective and anti seizure effects they may be useful in case of ictus or spinal or cerebral trauma;
- given their antinocicettive effects they may be used in association of pain-killer drugs such as opiods;
- given the bronchodilator capacity and anti-hypertensive action they may be useful in case of respiratory failure, heart deficiency and hypertension;
- given their capacity in inhibiting cell growth in vitro they may be useful in the therapy of cancer;
- given their capacity to stimulate food intake they may be useful in cachetic patients (e.g terminal phase AIDS syndrome or oncological patients);
- given their capacity to reduce the nausea and emesis they may be useful for treatment of nausea or emesis in patients that undergo chemiotherapy;
- given their capacity to inhibit the release of pro-inflammatory mediators from mast cells, they might be useful for treatment of allergic conditions (e.g. atopic dermatitis, asthma);
- given their capacity to lower the intra-ocular pressure they may be useful in subjects with glaucoma.

Only the treatment of dermatitis, contact and atopic dermatitis is part of the present invention.

The compounds of the present invention may be administered through different routes for preventive or therapeutic treatment of specific pathological condition; these administration routes include, in fact, oral, parenteral, intramuscular, subcutaneous, topical, transdermal, intravenous, rectal, sublingual, and intranasal. The compounds, according to the therapeutic use as cannabinoid-like molecules can be administered in pharmaceutical compositions in combination with excipients, dispersants and diluents compatible with the pharmaceutical uses known or even new, with the aim to obtain an improved delivery of the active ingredient to the site of action and to obtain a rapid, or a sustained or a delayed in time effect. The dosages are dependent on the severity of the pathology or disorder and on the chosen route of administration, as well as on the state (age, body weight, general health condition) of the patient. For illustrative purposes, but not limited to, the dosages of molecules included the present invention might range between 1 and 50 mg/kg in daily repeated or administration for a period ranging from 2 to 16 weeks.

For oral administration, compositions in the form of dispersible granular powders, tablets, pills, hard and soft gelatine capsules, suspensions, emulsions or solutions are suitable; for parenteral administration intramuscularly, subcutaneously, intravenously or peridurally, compositions in the form of buffered aqueous solutions, oil suspensions or lyophilised powders dispersible in appropriate solvents at the time of the administration can be suitable; for topical administration transdermally, rectally, intranasally or sublingually, composition in appropriate excipients or dispersants in the form of solutions, emulsions, suspensions, gels, ointments, creams, patches, suppositories, ovules, aerosols, sprays and tablets can be suitable.

## Claims

1. Compounds of formula (I)
R-CO-NH-R₁ (I)
where
R₁ is the chain of a primary amine selected from the group consisting of 2-amino-propane and cyclopropylamine; and
R is the alkyl residue of a carboxylic acid selected from the group consisting of myristic, palmitic, stearic and arachidic acids,
for use as active compounds in therapeutic or preventive treatment of pathological conditions or disorders usefully treatable with molecule having cannabinoid/endocannabinoid-like activity, wherein said pathologies are selected from the group consisting of dermatitis, contact dermatitis and atopic dermatitis.

2. The compound for use according to claim 1, wherein R is the alkyl residue of myristic acid and R₁ is the alkyl residue of 2-amino-propane.

3. The compound for use according to claim 1, wherein R is the alkyl residue of palmitic acid and R₁ is the alkyl residue of 2-amino-propane.

4. The compound for use according to claim 1, wherein R is the alkyl residue of stearic acid and R₁ is the alkyl residue of 2-amino-propane.

5. Use of the compounds according to claim 1 as active ingredient for the preparation in combination with pharmaceutically acceptable excipients and/or diluents of compositions for therapeutic or preventive treatment of pathological conditions or disorders usefully treatable with molecule having cannabinoid/endocannabinoid-like activity, wherein said pathologies are selected from the group consisting of dermatitis, contact dermatitis and atopic dermatitis.

6. The use according to claim 5, wherein said pharmaceutical compositions are suitable for oral administration in the form of dispersible granular powder, tablets, pills, soft or hard gelatine capsules, suspensions, emulsions and solutions.

7. The use according to claim 5, wherein said pharmaceutical compositions are suitable for administration parenterally, intramuscularly, subcutaneously, endovenously, in the form of buffered aqueous or oil solutions, aqueous or oil suspensions or lyophilized powder dispersible in appropriate solvents at the time of administration.

8. The use according to claim 5, wherein said pharmaceutical compositions are suitable for administration topically, transdermally, rectally, intranasally or sublingually, in appropriate excipients or dispersants in the form of solutions, emulsions, suspensions, gels, ointments, creams, patches, suppositories, ovules, aereosol, spray and tablets.

9. The use according to claim 5, wherein in said pathological states said therapeutic compositions comprising said compounds of formula (I) having cannabinoid/endocannabinoid-like activity are to be used alone or in association with other medications specific for the pathology or disorder to be treated.

## Patentansprüche

1. Verbindungen gemäß der Formel (I)
R-CO-NH-R₁ (I)
worin:
R₁ die Kette eines primären Amins ausgewählt aus der Gruppe bestehend aus 2-Aminopropan und Cyclopropylamin ist und
R der Alkylrest einer Carbonsäure ausgewählt aus der Gruppe bestehend aus Myristinsäure, Palmitinsäure, Stearinsäure und Arachidonsäure ist,
zur Verwendung als aktive Bestandteile bei der therapeutischen oder präventiven Behandlung von pathologischen Zuständen oder
Störungen, welche zweckdienlich mit einem Molekül mit einer Cannabinoid-/Endocannabinoid-ähnlichen Aktivität behandelt werden, wobei die Erkrankungen aus der Gruppe ausgewählt sind, welche aus Dermatitis, Kontaktdermatitis und atopischer Dermatitis besteht.

2. Verbindung zur Verwendung nach Anspruch 1, wobei R der Alkylrest von Myristinsäure ist und R₁ der Alkylrest von 2-Aminopropan ist.

3. Verbindung zur Verwendung nach Anspruch 1, wobei R der Alkylrest von Palmitinsäure ist und R₁ der Alkylrest von 2-Aminopropan ist.

4. Verbindung zur Verwendung nach Anspruch 1, wobei R der Alkylrest von Stearinsäure ist und R₁ der Alkylrest von 2-Aminopropan ist.

5. Verwendung der Verbindungen nach Anspruch 1 als aktiver Bestandteil in Mischung mit pharmazeutisch akzeptablen Hilfsstoffen und/oder Verdünnungsmitteln für die Herstellung von Zusammensetzungen zur therapeutischen oder präventiven Behandlung von pathologischen Zuständen oder Störungen, welche zweckdienlich mit einem Molekül mit einer Cannabinoid- /Endocannabinoid-ähnlichen Aktivität behandelt werden, wobei die Erkrankungen aus der Gruppe ausgewählt sind, welche aus Dermatitis, Kontaktdermatitis und atopischer Dermatitis besteht.

6. Verwendung nach Anspruch 5, wobei die pharmazeutischen Zusammensetzungen in der Form von dispergierbarem granulärem Pulver, Tabletten, Pillen, weichen oder harten Gelatinekapseln, Suspensionen, Emulsionen und Lösungen zur oralen Verabreichung geeignet sind.

7. Verwendung nach Anspruch 5, wobei die pharmazeutischen Zusammensetzungen zu dem Zeitpunkt ihrer Verabreichung in der Form von gepufferten wässrigen oder öligen Lösungen, wässrigen oder öligen Suspensionen oder lyophilisiertem Pulver, das in geeigneten Lösungsmitteln dispergierbar ist, zur parenteralen, intramuskulären, subkutanen, endovenösen Verabreichung geeignet sind.

8. Verwendung nach Anspruch 5, wobei die pharmazeutischen Zusammensetzungen in geeigneten Hilfsmitteln oder Dispergiermitteln in der Form von Lösungen, Emulsionen, Suspensionen, Gelen, Salben, Cremes, Pflastern, Suppositorien, Ovula, Aerosol, Spray und Tabletten zur topischen, transdermalen, rektalen, intranasalen oder sublingualen Verabreichung geeignet sind.

9. Verwendung nach Anspruch 5, wobei die therapeutischen Zusammensetzungen, welche Verbindungen gemäß der Formel (I) mit einer Cannabinoid-/Endocannabinoid-ähnlichen Aktivität enthalten, in den pathologischen Zuständen alleine oder in Mischung mit anderen Arzneimitteln, welche für die zu behandelnde Krankheit oder Störung spezifisch sind, einzusetzen sind.

## Revendications

1. Composés de formule (I)
**R-CO-NH-R₁** **(I)**
dans laquelle
R₁ est la chaîne d'une amine primaire choisie dans le groupe constitué par le 2-amino-propane et la cyclopropylamine ; et
R est le résidu alkyle d'un acide carboxylique choisi dans le groupe constitué par les acides myristique, palmitique, stéarique et arachidique,
à utiliser comme composés actifs dans le traitement thérapeutique ou préventif d'affections ou troubles pathologiques pouvant être utilement traités avec les molécules ayant une activité de type cannabinoïde/endocannabinoïde, lesdites pathologiques étant choisies dans le groupe constitué par la dermatite, la dermatite de contact et la dermatite atopique.

2. Composé à utiliser selon la revendication 1, dans lequel R est le résidu alkyle de l'acide myristique et R₁ est le résidu alkyle du 2-amino-propane.

3. Composé à utiliser selon la revendication 1, dans lequel R est le résidu alkyle de l'acide palmitique et R₁ est le résidu alkyle du 2-amino-propane.

4. Composé à utiliser selon la revendication 1, dans lequel R est le résidu alkyle de l'acide stéarique et R₁ est le résidu alkyle du 2-amino-propane.

5. Utilisation des composés selon la revendication 1 comme ingrédient actif pour la préparation en combinaison avec des excipients et/ou diluants pharmaceutiquement acceptables de compositions destinées à un traitement thérapeutique ou préventif d'affections ou troubles pathologiques pouvant être utilement traités avec une molécule ayant une activité de type cannabinoïde/endocannabinoïde, lesdites pathologiques étant choisies dans le groupe constitué par la dermatite, la dermatite de contact et la dermatite atopique.

6. Utilisation selon la revendication 5, dans laquelle lesdites compositions pharmaceutiques sont appropriées pour une administration orale sous la forme d'une poudre granulaire dispersible, de comprimés, de pilules, de gélules molles ou dures, de suspensions, d'émulsions et de solutions.

7. Utilisation selon la revendication 5, dans laquelle lesdites compositions pharmaceutiques sont appropriées pour une administration par voie parentérale, intramusculaire, sous-cutanée, endoveineuse, sous la forme de solutions aqueuses ou huileuses tamponnées, suspensions aqueuses ou huileuses ou d'une poudre lyophilisée dispersible dans des solvants appropriés au moment de l'administration.

8. Utilisation selon la revendication 5, dans laquelle lesdites compositions pharmaceutiques sont appropriées pour une administration par voie topique, transdermique, rectale, intranasale ou sublinguale, dans des excipients ou dispersants appropriés sous la forme de solutions, émulsions, suspensions, gels, pommades, crèmes, timbres, suppositoires, comprimés vaginaux, aérosols, sprays et comprimés.

9. Utilisation selon la revendication 5, dans laquelle, dans lesdits états pathologiques, lesdites compositions thérapeutiques comprenant lesdits composés de formule (I) ayant une activité de type cannabinoïde/endocannabinoïde doivent être utilisées seules ou en association avec d'autres médications spécifiques de la pathologique ou du trouble à traiter.
